# EUROPEAN PATENT APPLICATION

(11) **EP 3 139 261 A1**
(43) Date of publication of application: **08.03.2017**
(21) Application number: 16179624.8
(22) Date of filing: 15.07.2016
(51) Int. Cl.: G06F 3/0488, G06F 1/16, G06F 3/01, G06F 1/32, G06F 3/16, H04M 1/725, H04R 25/00

(54) **USER TERMINAL APPARATUS, SYSTEM, AND METHOD FOR CONTROLLING THE SAME**

(30) Priority: 03.09.2015 KR 20150124716
(71) Applicant: Samsung Electronics Co., Ltd., Suwon-si, Gyeonggi-do 16677 (KR)
(72) Inventor: KIM, Ji-hyae, Seoul (KR); JUN, Se-ran, Gyeonggi-do (KR); LEE, Won-hee, Seoul (KR)
(74) Representative: Appleyard Lees IP LLP

(57) **Abstract**

A user terminal apparatus is provided. The user terminal apparatus includes a communicator configured to communicate with an external apparatus; a detector configured to detect a touch operation of a user with respect to the user terminal apparatus; and a processor configured to control the communicator to generate a control command for controlling at least one of a plurality of speakers according to a type of the touch operation and configured to transmit the generated control command to the external apparatus.

## Description

### CROSS-REFERENCE TO RELATED APPLICATION

This application claims priority from Korean Patent Application No. 10-2015-0124716, filed on September 3, 2015, in the Korean Intellectual Property Office, the disclosure of which is incorporated herein in its entirety by reference.

### BACKGROUND

### 1. Field

Apparatuses and methods consistent with exemplary embodiments relate to a user terminal apparatus, a system, and a method for controlling the same, and more particularly, to a user terminal apparatus which generates a control command based on a user's interaction, a system, and a method for controlling the same.

### 2. Description of the Related Art

With the development of electronic technologies, various types of electronic apparatuses have been developed and widely used. For example, various display apparatuses, such as, a television (TV), a mobile phone, a personal computer (PC), a laptop PC, a personal digital Assistant (PDA), and the like, are widely used. In addition, a user terminal apparatus that may be comfortably wearable by a user is increasingly used. For example, the wearable user terminal apparatus may be provided in a form of eyeglasses, a watch, clothes, and the like.

Accordingly, more diverse functions of the user terminal apparatus may be provided. For example, a user terminal apparatus that is put on a user's body may collect, through various types of user's interactions, information such as biometric information or behavior information about a user to perform diverse functions based on the collected information.

In the wearable user terminal apparatus, there is a need for controlling a plurality of speakers through various user's manipulations with respect to the wearable user terminal apparatus and controlling a plurality of speakers connected with the same network through the wearable user terminal apparatus.

### SUMMARY

One or more exemplary embodiments provide a user terminal apparatus which generates and transmits a control command for controlling a plurality of speakers according to a type of a user's touch, a system, and a method for controlling the same.

According to an aspect of an exemplary embodiment, there is provided a user terminal apparatus including: a communicator configured to communicate with an external apparatus; a detector configured to detect a touch operation of a user with respect to the user terminal apparatus; and a processor configured to control the communicator to generate a control command for controlling at least one of a plurality of speakers according to a type of the touch operation and configured to transmit the generated control command to the external apparatus.

The apparatus may further include a display configured to display a user interface (UI) screen associated with the at least one of the plurality of speakers, wherein, in response to a touch operation in which a substantial portion of the UI screen is covered, the processor is configured to generate a control command for muting the at least one of the plurality of speakers and configured to transmit the generated control command to the external apparatus.

In response to a predetermined motion of the user being recognized within a predetermined distance from the UI screen, the processor may generate a control command for decreasing a volume level of the at least one of the plurality of speakers and transmit the generated control command to the external apparatus.

In response to a user manipulation for controlling the at least one of the plurality of speakers being received with respect to the UI screen, the processor may transmit a control command corresponding to the user manipulation to the external apparatus.

The apparatus may further include a sensor configured to sense location information of the user terminal apparatus, wherein in response to determining that at least one speaker among the plurality of speakers exists within a predetermined distance from the user terminal apparatus based on the location information of the user terminal apparatus, the processor is configured to automatically display a UI screen for controlling the at least one speaker.

The apparatus may further include a recognizer configured to recognize a voice of the user, wherein in response to a voice of the user for controlling the at least one of the plurality of speakers being recognized, the processor is configured to transmit, to the external apparatus, a control command for controlling the at least one of the plurality of speakers based on the recognized voice.

In response to the voice of the user for controlling the at least one of the plurality of speakers being recognized, the processer may transmit, to the external apparatus, a control command for retrieving, based on the recognized voice, an audio content and outputting the audio content through a speaker specified by the voice.

The sensor may sense at least one of time information and biometric information, wherein the processor is configured to transmit, to the external apparatus, a control command for specifying, based on the location information on the user terminal apparatus, the at least one of the plurality of speakers and retrieving, based on the at least one of the time information and the biometric information, an audio content.

In response to determining that a distance between the user terminal apparatus and the external apparatus exceeds a predetermined threshold value based on the location information on the user terminal apparatus, the processor may transmit a control command for turning off the plurality of speakers connected with the external apparatus, to the external apparatus.

According to an aspect of another exemplary embodiment, there is provided a system including: a plurality of network speakers; a first terminal apparatus configured to transmit a control command for controlling at least one of the plurality of network speakers; and a second terminal apparatus configured to receive the control command and control the at least one of the plurality of network speakers based on the received control command, wherein the first terminal apparatus is configured to detect a touch operation of a user with respect to the first terminal apparatus and generate the control command for controlling the at least one of the plurality of network speakers according to a type of the touch operation.

The first terminal apparatus may display a user interface (UI) screen for controlling the at least one of the plurality of network speakers, and in response to a touch operation of covering the entire UI screen being detected, the first terminal apparatus may generate a control command for muting the at least one of the plurality of network speakers, and transmit the generated control command to the second terminal apparatus.

In response to a predetermined user's motion being recognized within a predetermined distance from the UI screen, the first terminal apparatus may generate a control command for decreasing a volume level of the at least one of the plurality of network speakers and transmit the generated control command to the second terminal apparatus.

According to an aspect of still another exemplary embodiment, there is provided a method for controlling a user terminal apparatus which communicates with an external apparatus that controls a plurality of speakers, the method including: detecting a touch operation of a user; and generating a control command for controlling at least one of the plurality of speakers according to a type of the touch operation and transmitting the generated control command to the external apparatus.

The method may further include displaying a user interface (UI) screen for controlling the at least one of the plurality of speakers, wherein in response to a touch operation of covering the entire UI screen being detected, the transmitting includes generating a control command for muting the at least one of the plurality of speakers and transmitting the generated control command to the external apparatus.

In response to a predetermined motion of the user being recognized within a predetermined distance from the UI screen, the transmitting may include generating a control command for decreasing a volume level of the at least one of the plurality of speakers and transmitting the generated control command to the external apparatus.

In response to a manipulation of the user for controlling the at least one of the plurality of speakers being received with respect to the UI screen, the transmitting may include transmitting a control command corresponding to the manipulation to the external apparatus.

The method may further include sensing location information on the user terminal apparatus, wherein in response to determining that at least one speaker among the plurality of speakers exists within a predetermined distance from the user terminal apparatus based on the location information on the user terminal apparatus, the displaying includes automatically displaying a UI screen for controlling the at least one speaker.

The method may further include recognizing a voice of the user, wherein in response to a voice of the user for controlling the at least one of the plurality of speakers being recognized, the transmitting includes transmitting a control command corresponding to the voice to the external apparatus.

In response to the voice being recognized, the transmitting may include transmitting a control command for retrieving an audio content based on the voice and outputting the audio content through a speaker specified by the voice to the external apparatus.

The method may further include sensing at least one of time information and biometric information, wherein the transmitting includes transmitting, to the external apparatus, a control command for specifying, based on the location information on the user terminal apparatus, the at least one of the plurality of speakers and retrieving, based on at least one of the time information and the biometric information, an audio content.

According to an aspect of still another exemplary embodiment, there is provided a wearable device including: a display; a communicator configured to communicate with an external apparatus; a sensor configured to sense information including at least one of location information indicating a location of the wearable device, time information indicating a current time, and biometric information of a user who wears the wearable device; a processor configured to generate a control command for controlling at least one of a plurality of speakers based on the sensed information and transmit the control command to the external apparatus.

The sensor may sense the location information, and the processor may determine the at least one of the plurality of speakers based on the location information and control the display to display a user interface (UI) configured to receive a user input to control the at least one of the plurality of speakers.

The sensor may sense the time information, and the processor may determine an audio content based on the time information and transmit a control command for controlling the at least one of the plurality of speakers to reproduce the audio content, to the external apparatus.

The sensor may sense the biometric information, and the processor may determine an audio content based on the biometric information and transmit a control command for controlling the at least one of the plurality of speakers to reproduce the audio content, to the external apparatus.

The biometric information may include at least one of a brainwave, an electromyogram (EMG), and an electrocardiogram (ECG).

### BRIEF DESCRIPTION OF THE DRAWINGS

The above and/or other aspects will be more apparent by describing certain example embodiments with reference to the accompanying drawings, in which:
FIG. 1 is a block diagram illustrating a structure of a user terminal apparatus according to an exemplary embodiment;
FIG. 2 is a block diagram illustrating a structure of a user terminal apparatus according to another exemplary embodiment;
FIG. 3 is a view illustrating a user interface (UI) screen according to an exemplary embodiment;
FIGS. 4 and 5 are views illustrating a user's interaction with a user terminal apparatus according to exemplary embodiments;
FIGS. 6 to 13 are views illustrating various UI screens according to exemplary embodiments;
FIG. 14 is a block diagram illustrating a structure of a user terminal apparatus according to another exemplary embodiment;
FIG. 15 is a view illustrating a speaker control screen displayed based on location information of a user terminal apparatus according to an exemplary embodiment;
FIG. 16 is a block diagram illustrating a structure of a user terminal apparatus according to still another exemplary embodiment;
FIG. 17 is a view illustrating biometric information according to an exemplary embodiment;
FIG. 18 is a view illustrating a user terminal apparatus including various sensors according to an exemplary embodiment;
FIG. 19 is a block diagram illustrating a structure of a system according to an exemplary embodiment;
FIGS. 20A and 20B are views illustrating a process of adjusting a volume level of a plurality of network speakers in a television (TV) according to an exemplary embodiment;
FIG. 21 is a block diagram illustrating a detailed structure of the user terminal apparatus of FIG. 1;
FIG. 22 is a view illustrating software modules stored in a storage according to an exemplary embodiment; and
FIG. 23 is a flowchart illustrating a method for controlling a user terminal apparatus according to an exemplary embodiment.

### DETAILED DESCRIPTION

Certain exemplary embodiments are described in greater detail below with reference to the accompanying drawings. In the following description, like drawing reference numerals are used for the like elements, even in different drawings. The matters defined in the description, such as detailed construction and elements, are provided to assist in a comprehensive understanding of exemplary embodiments. However, exemplary embodiments can be practiced without those specifically defined matters. Also, well-known functions or constructions are not described in detail since they would obscure the application with unnecessary detail.

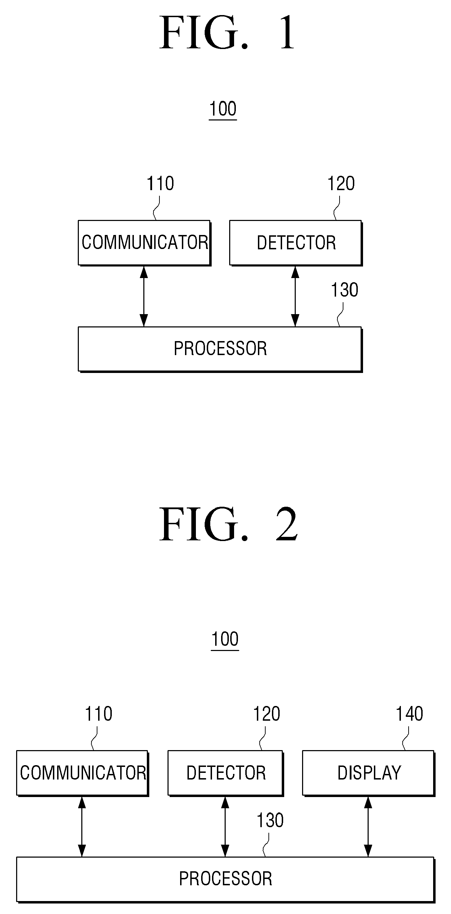

FIG. 1 is a block diagram illustrating a structure of a user terminal apparatus according to an exemplary embodiment.

Referring to FIG. 1, a user terminal apparatus 100 includes a communicator 110, a detector 120, and a processor 130. The user terminal apparatus 100 according to an exemplary embodiment may include a wearable device which operates via wireless communication with an external apparatus such as a smart phone, a tablet personal computer (PC), and the like.

The user terminal apparatus 100 according to an exemplary embodiment will be described in the following description by taking an example of a smart watch among various types of electronic apparatuses. However, exemplary embodiments are not limited to this and operations of the user terminal apparatus 100 according to exemplary embodiments may be applied to various types of wearable devices.

The communicator 110 may communicate with an external apparatus that is capable of controlling a plurality of speakers. Herein, the external apparatus refers to an electronic apparatus which is connected with a plurality of speakers through the same network, that is, the same access point (AP), and is capable of controlling the plurality of speakers. For example, the external apparatus may be provided as a television (TV), a laptop PC, a tablet PC, a desktop PC, a set-top box, a game console, a stereo system, a mobile phone, and the like.

The communicator 110 may communicate with the external apparatus according to diverse communication methods, such as, Bluetooth (BT), wireless fidelity (Wi-Fi), Zigbee, infrared rays (IR), serial interface, universal serial bus (USB), near field communication (NFC), and the like.

The external apparatus may be classified into a stationary apparatus and a movable apparatus. The stationary apparatus may include a TV, a desktop PC, a set-top box, a game console, and a stereo system, and the movable apparatus may include a laptop PC, a tablet PC, and a mobile phone in the aforementioned examples.

The plurality of speakers may be connected with the external apparatus in a wired communication manner and/or in a wireless communication manner. The plurality of speakers may be arranged in one place or may be arranged a certain distance apart from each other.

The detector 120 may detect a user's touch on the user terminal apparatus 100. The detector 120 may be provided as a touch panel for detecting a user's touch manipulation. The touch panel may be embedded in a display and a bezel of a smart watch.

The processor 130 may control the communicator 110 to generate a control command for controlling the plurality of speakers and transmit the generated control command to the external apparatus according to a type of the user's touch operation.

In an exemplary embodiment, the processor 130 may generate a control command corresponding to the user's touch and transmit the generated control command to the external apparatus. The external apparatus may control the plurality of speakers based on the received control command.

FIG. 2 is a block diagram illustrating a structure of a user terminal apparatus according to another exemplary embodiment.

Referring to FIG. 2, a user terminal apparatus 100 includes a communicator 110, a detector 120, a processor 130, and a display 140. The communicator 110, the detector 120, and the processor 130 may have the same or similar structure to those that have been described above, and thus, a detailed description thereof will be omitted.

The display 140 may display a user interface (UI) screen for controlling at least one of a plurality of speakers. In addition, the display 140 may display diverse objects, such as, a still image, a picture, a document, and the like. Accordingly, the display 140 may be provided as a liquid crystal display (LCD), an organic light-emitting diode (OLED), a plasma display panel (PDP), and the like.

The processor 130 may display a UI screen for controlling at least one of the plurality of speakers through the display 140. As an example, when there are a plurality of speakers including Speaker 1, Speaker 2, and Speaker 3, the processor 130 may display a UI screen for controlling all of the Speakers 1 to 3, display a UI screen for controlling only the Speakers 1 and 2, or display a UI screen for controlling only the Speaker 1.

In an exemplary embodiment, in response to a user's touch being detected with respect to the UI screen for controlling all of the Speakers 1 to 3, the processor 130 may transmit a control command for controlling all of the Speakers 1 to 3 to the external apparatus according to the detected user's touch.

In addition, the processor 130 may display the UI screen for controlling only the Speakers 1 and 2 on the display 140 in response to a user's manipulation. In response to a user's touch being detected with respect to the UI screen for controlling only the Speakers 1 and 2, the processor 130 may transmit a control command for controlling only the Speaker 1 and 2 to the external apparatus.

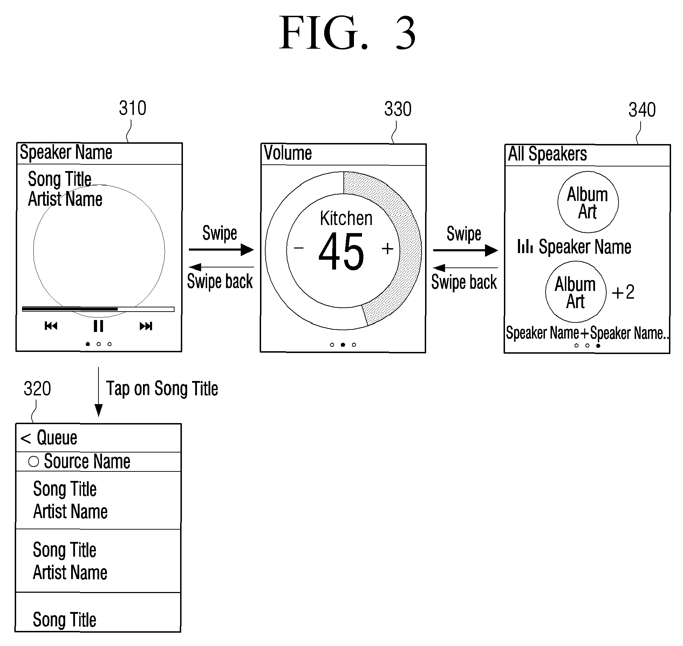

Hereinafter, a UI screen which is configured to receive a user input (e.g., user's touch) to control a plurality of speakers and is displayed on the display 140 will be described with reference to FIG. 3.

FIG. 3 is a view illustrating a UI screen according to an exemplary embodiment.

Referring to FIG. 3, the processor 130 may control to display an audio content reproduction screen 310 on the display 140. In this case, the audio content reproduction screen 310 may display a name of a speaker (or speaker name), a name of an audio content (or song title), a name of a singer (or artist's name), and icons representing diverse functions related to controlling the audio content (for example, reproduce a previous song, reproduce a next song, pause, and the like).

In an exemplary embodiment, in response to a user's touch being detected with respect to a name of an audio content (or song title), the processor 130 may control to display an audio content reproduction list 320 on the display 140. In addition, in response to an audio content different from an audio content which is currently being reproduced being selected by a user's touch from the audio content reproduction list 320, the processor 130 may generate a control command for displaying a reproduction screen for the selected audio content on the display 140 and reproducing the selected audio content and transmit the control command to the external apparatus.

In response to a user's touch operation for changing a screen being detected with respect to the audio content reproduction screen 310, the processor 130 may change the audio content reproduction screen 310 to an audio content-volume level adjustment screen 330 and display the screen 330 on the display 140. In this case, the user's touch operation for changing a screen may be a manipulation of a touch and drag, e.g., dragging a pre-executed user's touch to the left or to the right on the display 140. For example, in response to a user's manipulation of dragging a pre-executed user's touch to the left, the processor 130 may change the audio content reproduction screen 310 to the audio content-volume level adjustment screen 330 with a graphical effect that the audio content reproduction screen 310 is pushed to the left. Also, in response to the audio content-volume level adjustment screen 330 being displayed in the display 140 and a user's manipulation of dragging a pre-executed user's touch to the right being detected, the processor 130 may change the audio content-volume level adjustment screen 330 to the audio content reproduction screen 310 which was previously displayed with a graphical effect that the audio content-volume level adjustment screen 330 is pushed to the right.

The audio content-volume level adjustment screen 330 may display a name (e.g., "Kitchen") of a speaker to inform a user of a location of the speaker and an icon corresponding to a volume of a displayed speaker. Referring to FIG. 3, the audio content-volume level adjustment screen 330 displays an icon corresponding to a function of adjusting a volume of a speaker located at a kitchen. In response to a user's manipulation of changing a name of the speaker being detected, the processor 130 may control to display an icon corresponding to a function of adjusting a volume of a speaker having a changed name.

In response to the audio content-volume level adjustment screen 330 being displayed on the display 140 and a user's manipulation of dragging a pre-executed user's touch to the left being detected, the processor 130 may change the audio content-volume level adjustment screen 330 to a speaker list screen 340 with a graphical effect that the audio content-volume level adjustment screen 330 is pushed to the left.

The speaker list screen 340 may display a plurality of speakers connected with an access point which is the same as that of the external apparatus and display which speaker currently outputs an audio content. In addition, the speaker list screen 340 may group and display at least two or more speakers among the plurality of speakers into one group. For example, the processor 130 may group and display a first speaker located at Bedroom 1 and a second speaker located at Bedroom 2 into a first group. In response to a user's touch of adjusting a volume of the speakers or changing an audio content being detected with respect to the first group, the processor 130 may transmit a control command for controlling the volume of the first and second speakers or outputting a changed audio content through the first and second speakers to the external apparatus.

The processor 130 may group and display at least two or more speakers into one group according to a user's manipulation. For example, icons corresponding to each of a first speaker at a living room, a second speaker at a kitchen, and a third speaker at a bedroom are displayed on the display 140 and in response to a user's manipulation of selecting the icons (e.g., gathering the icons into one place) being detected, the processor 130 may group the first, second, and third speakers into one group and display an icon corresponding to the group in a form in which the icons corresponding to each of the first, second, and third speakers are included.

In response to a user's manipulation of controlling a volume of the speakers or changing an audio content being detected with respect to the icon corresponding to the group while the icon corresponding to the group is displayed, the processor 130 may transmit a control command for controlling a volume of the speakers or changing an audio content to the external apparatus, with respect to all of the grouped first, second, and third speakers, as described above.

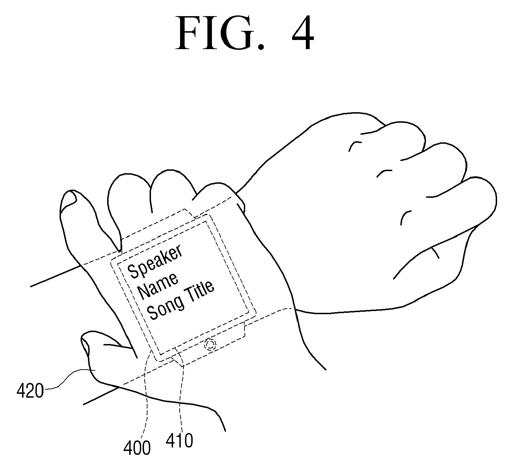

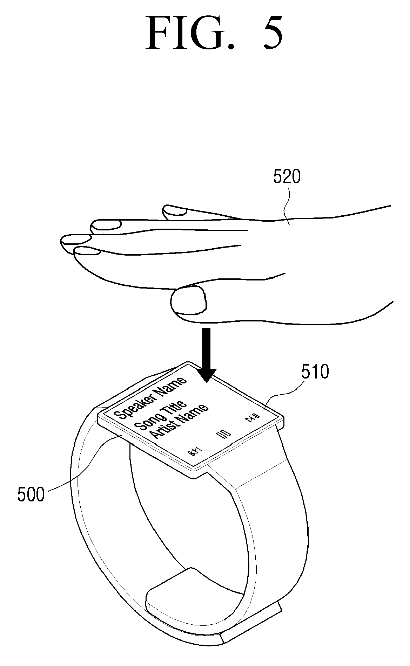

The user terminal apparatus according to an exemplary embodiment may be a wearable device. In this case, a user may easily perform interaction with respect to the user terminal apparatus. For example, in response to a predetermined user's interaction being performed with respect to a UI screen displayed on the display 140, the processor 130 may perform a function corresponding to the user's interaction. FIGS. 4 and 5 are views illustrating a user's interaction with a user terminal apparatus according to exemplary embodiments.

Referring to FIG. 4, a user's touch of covering a screen 410 of a smart watch 400 by a user's hand 420 is performed.

In response to a user's touch of covering a part or an entirety of the UI screen 410 being detected, the processor 130 may generate a control command for muting at least one of a plurality of speakers and transmit the generated control command to the external apparatus.

In an exemplary embodiment, a UI screen for controlling a first speaker and a second speaker among a plurality of speakers is displayed, and in response to a user's touch of covering the entire UI screen (or a substantial amount of the UI screen) for controlling the first speaker and the second speaker being detected in this state, the processor 130 may generate a control command for muting the first and second speakers and transmit the generated control command to the external apparatus.

For example, a UI screen for controlling a speaker at a living room and a speaker at a kitchen among a plurality of speakers is displayed, and in response to a user's touch of covering the entire UI screen for controlling the speakers at the living room and kitchen being detected, the processor 130 may generate a control command for muting the speakers at the living room and kitchen and transmit the generated control command to the external apparatus. That is, a speaker which is not located at the living room and kitchen, for example, a speaker at a bed room or a speaker at a bathroom among the plurality of speakers is not muted.

In addition, a UI screen for controlling all of a plurality of speakers is displayed, and in response to a user's touch of covering the entire UI screen for controlling all of the plurality of speakers being detected, the processor 130 may generate a control command for muting all of the plurality of speakers and transmit the generated control command to the external apparatus.

In detecting a user's touch of covering the entire UI screen 410 displayed on the smart watch 400, the processor 130 may determine whether the user's touch is a touch of covering the entire UI screen by using a light quantity sensor and/or an illuminance sensor.

In an exemplary embodiment, in response to a user's touch of covering the UI screen 410 displayed on the smart watch 400 and covering an area greater than an area defined by a bezel by a predetermined degree being detected, the processor 130 may determine that a user's touch of covering the entire UI screen 410 is detected.

For example, in response to a user's touch of covering the UI screen 410 being detected with respect to the UI screen 410 displayed on the smart watch 400 simultaneously with detecting a user's touch of covering an area of more than 80 percent of the bezel area, the processor 130 may determine that the detected user's touch is a user's touch of covering the entire UI screen.

In response to determining that a level of light of the UI screen 410 of the smart watch 400 is similar to a predetermined threshold value by using the light quantity sensor and/or the illuminance sensor, the processor 130 may determine that a user's touch of covering the entire UI screen 410 is detected. For example, the predetermined threshold value may be zero.

In a case of using the light quantity sensor and/or illuminance sensor, FIG. 4 illustrates an example where a user covers the entire UI screen 410 displayed on the smart watch 4000 with the hand 420. However, exemplary embodiments are not limited to this and any object other than the hand 420 may be used to cover the entire UI screen 410.

Accordingly, the user may mute a particular speaker by performing a user's touch of covering the entire UI screen 410 for controlling a particular speaker of which the UI screen 410 is currently displayed on the smart watch 400.

In addition, in response to a predetermined user's motion being recognized within a predetermined distance from a UI screen, the processor 130 may generate a control command for decreasing a volume level of at least one of a plurality of speakers and transmit the generated control command to the external apparatus.

Referring to FIG. 5, a UI screen 510 for controlling an audio content is displayed on a smart watch 500, and a hand 520 approaches the UI screen 510 within a predetermined distance from the UI screen 510.

In response to a user's motion of moving the hand 520 to the UI screen 510 within a predetermined distance from the UI screen 510 being recognized while the UI screen 510 for controlling an audio content is displayed on the smart watch 500, the processor 130 may generate a control command for decreasing a volume level of a speaker of which the UI screen 510 is currently displayed and transmit the generated control command to the external apparatus.

For example, in response to a motion of moving the hand 520 to the UI screen 510 within a range of five centimeters from the UI screen 510 being recognized while the smart watch 500 displays the UI screen 510 indicating that a speaker at a kitchen reproduces an audio content, the processor 130 may generate a control command for decreasing a volume level of the speaker at the kitchen and transmit the generated control command to the external apparatus.

In addition, in response to a motion of moving the hand 520 to the UI screen 510 within the range of five centimeters from the UI screen 510 being recognized while the smart watch 500 displays the UI screen 510 indicating that the speaker at the kitchen and a speaker at a living room are grouped into one group and reproduce an audio content, the processor 130 may generate a control command for decreasing a volume level of the speakers at the kitchen and the living room and transmit the generated control command to the external apparatus.

The processor 130 may increase a speed of decreasing a volume level of a speaker displayed on the UI screen 510 based on a speed of moving the hand 520 to the UI screen 510 within a predetermined distance from the UI screen 510 and/or based on a distance between the UI screen 510 and the hand 520.

For example, the speaker at the kitchen reproduces an audio content, and in response to the speed of moving the hand 520 to the UI screen 510 within the range of five centimeters from the UI screen 510 being increased, the processor 130 may control the speed of decreasing the volume level of the speaker at the kitchen accordingly. In addition, when the hand 520 approaches the UI screen 510 and the distance between the hand 520 and the 510 decreases, the processor 130 may increase the speed of decreasing the volume level of the speaker at the kitchen.

In response to a motion of withdrawing the hand 520 from the UI screen 510 within a predetermined distance from the UI screen 510 being recognized, the processor 130 may generate a control command for increasing a volume level of a speaker of which the UI screen 510 is currently displayed and transmit the generated control command to the external apparatus.

In response to a user's manipulation of controlling at least one of a plurality of speakers being received with respect to a UI screen, the processor 130 may transmit a control command corresponding to the user's manipulation to the external apparatus.

In an exemplary embodiment, in response to a user's manipulation of controlling a speaker displayed on a UI screen being received, the processor 130 may transmit a control command corresponding to the user's manipulation to the external apparatus, and the external apparatus may control the speaker displayed on the UI screen based on the received control command. In this case, the speaker displayed on the UI screen may be at least one or more speakers.

The UI screen may include a screen for connecting communication with an external application, a screen for controlling a volume level of an audio content, a screen for selecting an audio content, and the like. In response to a user's manipulation being received with respect to respective screens of the UI screen, the processor 130 may generate a control command corresponding to the user's manipulation for each screen and transmit the generated control command to the external apparatus.

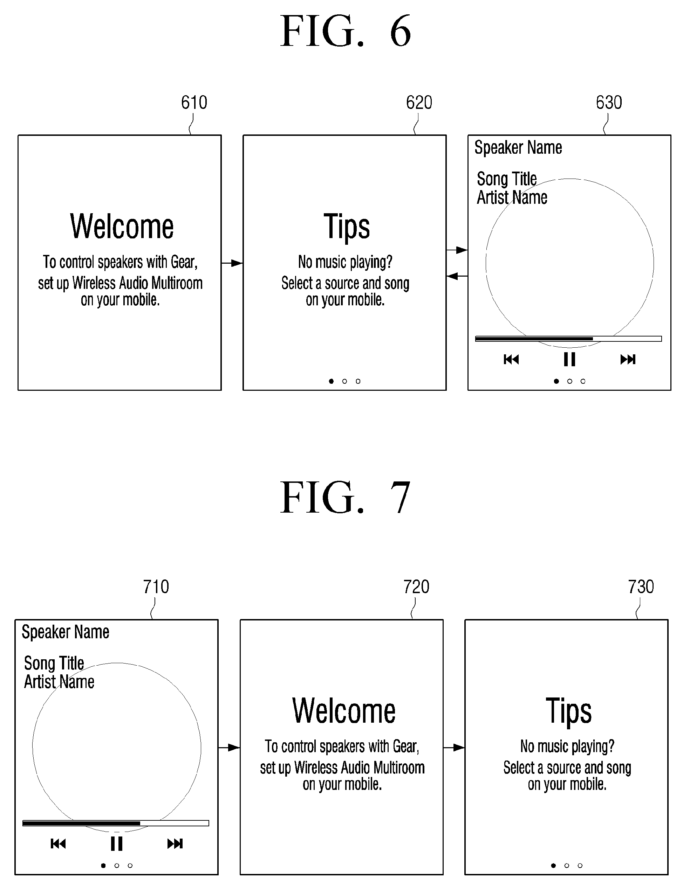

FIGS. 6 to 13 are views illustrating various UI screens of the user terminal apparatus according to exemplary embodiments.

FIG. 6 illustrates an initial screen 610 which is displayed on the display 140 in response to an application for controlling a plurality of speakers being executed in a user terminal apparatus 100. In an exemplary embodiment, the initial screen 610 may be displayed when the user terminal apparatus is not connected with the external apparatus.

In this case, in response to a user's manipulation of connecting communication with an external apparatus being performed by a user, the processor 130 may control a communicator 110 to connect the communication with the external apparatus. Subsequently, the processor 130 may display a screen 620 for configuring an audio content list.

In response to a user's manipulation of selecting audio contents and configuring an audio content list being received and one audio content being subsequently selected from the audio content list, the processor 130 may display an audio content control screen 630 including information on a name of a speaker outputting the selected audio content, a name of the audio content, and an artist's name.

In response to a user's touch with respect to the name of the audio content on the audio content control screen 630 being detected, the processor 130 may display the name of the audio content, for example, a screen including the audio content list in which the name of the audio content is shown. In response to a user's touch with respect to an icon for reproducing a previous song being detected, the processor 130 may reproduce a previous audio content, which precedes the audio content which is currently being reproduced in the audio content list. In response to a user's touch with respect to an icon for reproducing a next song being detected, the processor 130 may reproduce a next audio content, which follows the audio content which is currently being reproduced in the audio content list. In addition, in response to a user's touch with respect to a pause icon being detected, the processor 130 may pause reproduction of the audio content which is currently being reproduced.

FIG. 7 illustrates an example where connection between a user terminal apparatus 100 and an external apparatus is disconnected.

In an exemplary embodiment, in response to communication between the user terminal apparatus 100 and the external apparatus being disconnected from each other while a screen 710 for controlling an audio content which is currently being reproduced is displayed on the display 140, the processor 130 may display an initial screen 720 as in FIG. 6. The initial screen 720 is a screen displayed when the communication between the user terminal apparatus 100 and the external apparatus is not connected.

In response to a user's manipulation of connecting communication with the external apparatus being performed by a user while the initial screen 720 is displayed, the processor 130 may control a communicator 110 to connect the communication with the external apparatus. Subsequently, the processor 130 may display a screen 730 for configuring an audio content list.

That is, in response to the communication between the user terminal apparatus 100 and the external apparatus being disconnected from each other, the processor 130 may change and display the screen 710 for controlling the audio content which is currently being reproduced to the initial screen 720 such that the user is informed that connecting the communication between the user terminal apparatus 100 and the external apparatus needs to be performed to control the audio content.

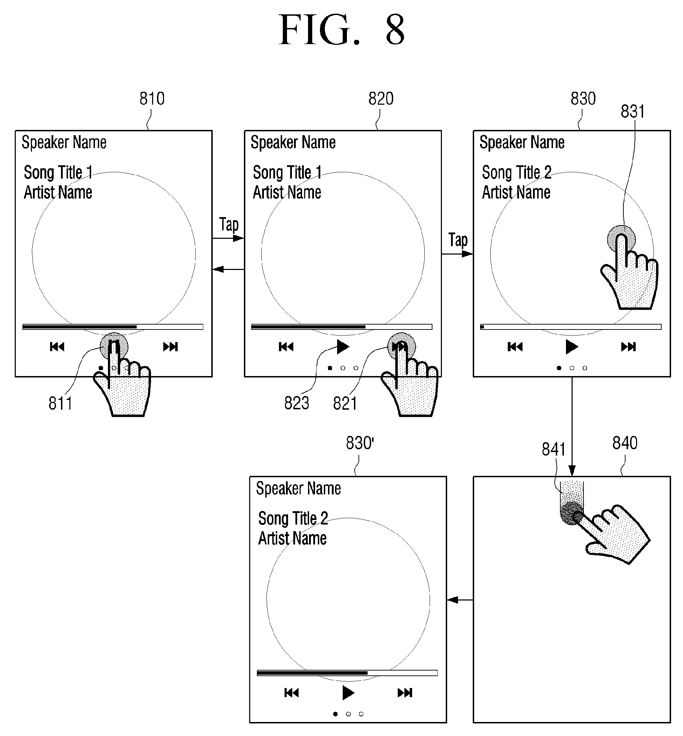

FIG. 8 illustrates UI screens for controlling audio content according to an exemplary embodiment.

Referring to FIG. 8, in response to a user's touch 811 with respect to a pause icon being detected while a screen 810 for controlling an audio content (Song Title 1) which is currently being reproduced is displayed, the processor 130 may generate a control command for stopping reproduction of the audio content (Song Title 1), transmit the generated control command to an external apparatus, and display the screen 820 where the pause icon has been changed to a reproduce icon 823.

In this case, in response to a user's touch with respect to the reproduce icon 823 being performed by a user, the processor 130 may generate a control command for resuming the reproduction of the audio content (Song Title 1), transmit the generated control command to the external apparatus, and change and display the screen 820 to the screen 810 for controlling the audio content (Song Title 1).

In response to a user's touch 821 with respect to an icon for reproducing a next song being detected while the screen 820 where the pause icon has been changed to the reproduce icon 823 is displayed, the processor 130 may generate a control command for outputting a next song (Song Title 2), which follows the audio content which is currently being reproduced in the audio content list, transmit the generated control command to the external apparatus, and display a screen 830 for controlling the next song (Song Title 2).

In response to a user's touch 831 with respect to a background area of the screen 830 for controlling the next song (Song Title 2) being detected while the screen 830 for controlling the next song (Song Title 2) is displayed, the processor 130 may display an album image 840 corresponding to the next song (Song Title 2) in a full screen.

In addition, in response to a user's touch 841 at or near a bezel area of the display 140 being detected while the album image 840 corresponding to the next song (Song Title 2) is displayed on a full screen, the processor 130 may display a screen 830' for controlling the next song (Song Title 2).

FIG. 8 illustrates an example where a user's touch at or near the bezel area of the display 140 is detected, however, the exemplary embodiments are not limited thereto. For example, the processor 130 may also display the screen 830' for controlling the next song (Song Title 2) in response to a user's touch with respect to only the bezel area.

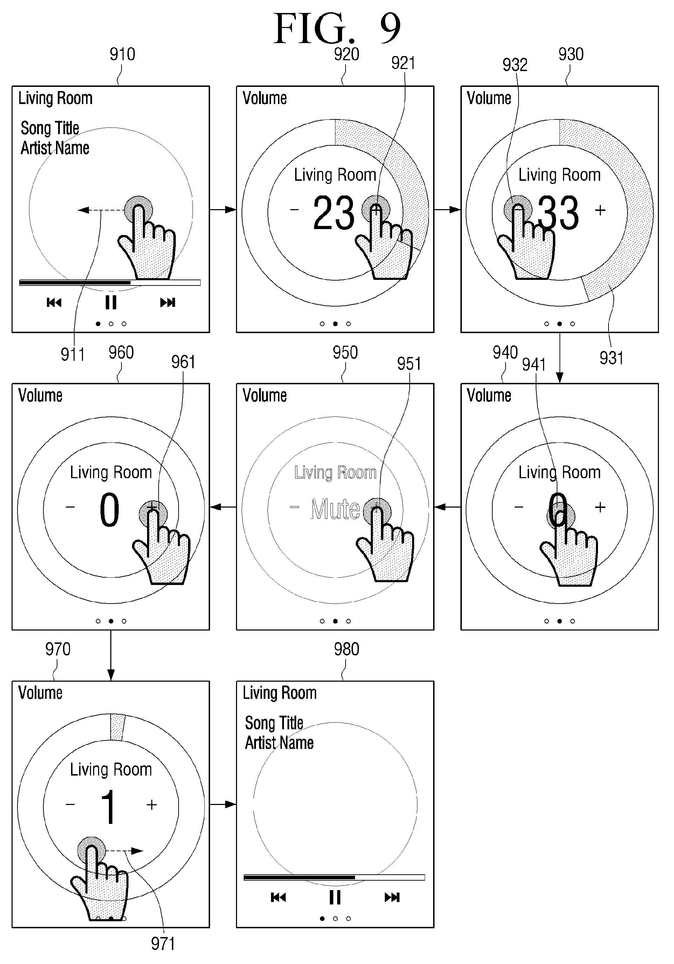

FIG. 9 illustrates a process of adjusting a volume level of a plurality of speakers according to an exemplary embodiment.

In an exemplary embodiment, in response to a user's manipulation 911 of dragging a pre-executed user's touch being detected while a screen 910 for controlling an audio content which is outputted through a speaker at a living room is displayed, the processor 130 may display a screen 920 for adjusting a volume level of the speaker at the living room. In this case, the screen 920 for adjusting a volume level of the speaker at the living room includes a current volume level of the speaker at the living room, an icon '+' and icon '-' for controlling the volume level, a circular bar-shaped icon corresponding to the volume level. That is, in response to a user's touch on the '+' icon, the volume level of the speaker at the living room increases, and a portion of the circular bar-shaped icon that is in a predetermined color increases to correspond to the increased volume level.

For example, as shown in FIG. 9, in response to a user's touch 921 with respect to the '+' icon for increasing a volume level being detected while the screen 920 for adjusting the volume level of the speaker at the living room is displayed, the processor 130 may generate a control command for increasing the volume level of the speaker at the living room from level of 23 to level of 33, transmit the generated control command to the external apparatus, and display a screen 930 including text representing that the volume level has been increased to level of 33 and a circular bar-shaped icon 931 having an increased portion in a predetermined color to correspond to the increased volume level.

In response to a user's touch 932 with respect to the '-' icon for decreasing a volume level being detected while the screen 930 including the text representing that the volume level has been increased to level of 33 and the circular bar-shaped icon 931 having an increased portion in the predetermined color to correspond to the increased volume level is displayed, the processor 130 may generate a control command for decreasing the volume level of the corresponding speaker, transmit the generated control command to the external apparatus, and display a screen 940 including text representing that the volume level has been decreased to, for example, level of 0 and a circular bar-shaped icon in which a portion in a predetermined color is not displayed to correspond to the volume level decreased to level of 0.

In this case, in response to a user's touch 941 with respect to the text representing that the volume level has been decreased to level of 0 being detected, the processor 130 may generate a control command for muting the speaker at the living room, transmit the generated control command to the external apparatus, and display a screen 950 representing that the speaker at the living room is in a muted state. The screen 950 representing that the speaker at the living room is in the muted state may be indicated in a predetermined color.

In response to a user's touch 951 with respect to the '+' icon for increasing a volume level being detected while the screen 950 representing that the speaker at the living room is in the muted state is displayed, the processor 130 may display a screen 960 for activating the volume level of the speaker at the living room. The screen 960 for activating the volume level of the speaker at the living room may display the text representing that the volume level of the speaker at the living room is level of 0 in a predetermined color.

In response to a user's touch 961 with respect to the '+' icon for increasing the volume level being detected on the screen 960 for activating the volume level of the speaker at the living room, the processor 130 may generate a control command for increasing the volume level of the speaker at the living room, transmit the generated control command to the external apparatus, and display a screen 970 including text representing that the volume level has been increased to level of 1 and a circular bar-shaped icon having a portion in a predetermined color to correspond to the increased volume level.

In response to a user's manipulation 971 of dragging a pre-executed user's touch to the right being detected while the screen 970 including the text representing that the volume level has been increased to level of 1 and the circular bar-shaped icon having a portion in the predetermined color to correspond to the increased volume level is displayed, the processor 130 may display a screen 980 for controlling an audio content which is being reproduced through the speaker at the living room. In this case, a volume level of the reproduced audio content may have a value of 1 as set in the screen 970.

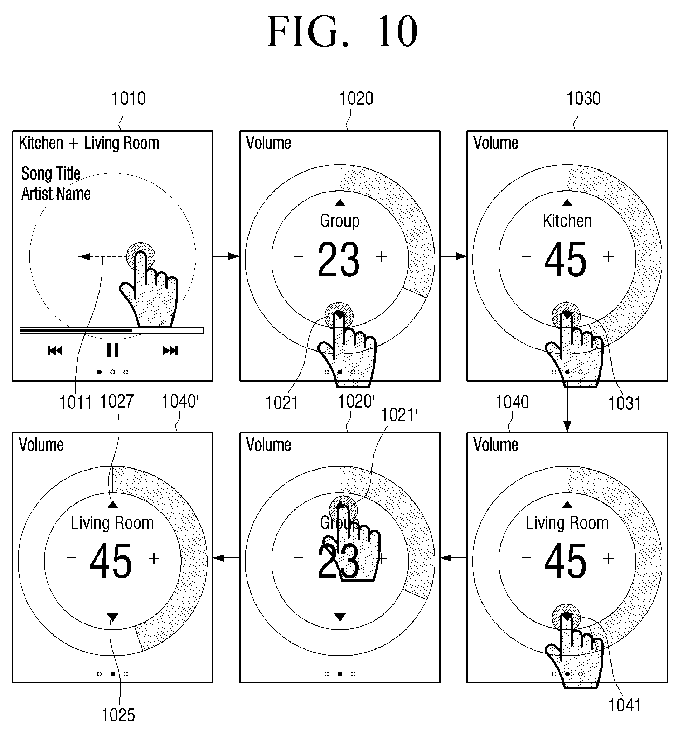

FIG. 10 illustrates a process of adjusting a volume level of a group including at least two or more speakers according to an exemplary embodiment.

In an exemplary embodiment, in response to a user's manipulation 1011 of dragging a pre-executed user's touch being detected while a screen 1020 for controlling an audio content which is being outputted through a speaker at a kitchen and a speaker at a living room is displayed, the processor 130 may display a screen 1020 for adjusting the volume level of the speakers at the kitchen and the living room. In this case, the screen 1020 for adjusting the volume level of the speakers at the kitchen and the living room includes text representing an integrated volume level of the group including the speakers at the kitchen and the living room, a '+' icon and a '-' icon for adjusting the integrated volume level, and a circular bar-shaped icon indicating the integrated volume level. That is, in response to the '+' icon being touched by a user, the integrated volume level of the group including the speakers at the kitchen and the living room increases, and the circular bar-shaped icon has an increased portion in a predetermined color to correspond to the increased integrated volume level.

In response to a user's touch 1021 with respect to a downward arrow-shaped icon (e.g., 1025 as shown in a screen 1040') being detected while the screen 1020 for adjusting the integrated volume level of the speakers at the kitchen and the living room is displayed, the processor 130 may display a screen 1030 for controlling the volume level of the speaker at the kitchen.

In response to a user's touch 1031 with respect to the downward arrow-shaped icon being detected while the screen 1030 for controlling the volume level of the speaker at the kitchen is displayed, the processor 130 may display a screen 1040 for controlling the volume level of the speaker at the living room.

In response to a user's touch 1040 with respect to the downward arrow-shaped icon being detected while the screen 1040 for controlling the volume level of the speaker at the kitchen is displayed, the processor 130 may display a screen 1020' for adjusting the integrated volume level of the group including the speakers at the kitchen and the living room. In addition, in response to a user's touch 1021' with respect to an upward arrow-shaped icon (e.g., 1027 as shown in the screen 1040') being detected while the screen 1020' for adjusting the integrated volume level of the group including the speakers at the kitchen and the living room is displayed, the processor 130 may display the screen 1040' for controlling the volume level of the speaker at the living room. That is, the user may touch the downward arrow-shaped icon or upward arrow-shaped icon to display the screens for individually or collectively controlling the respective speakers included in the group.

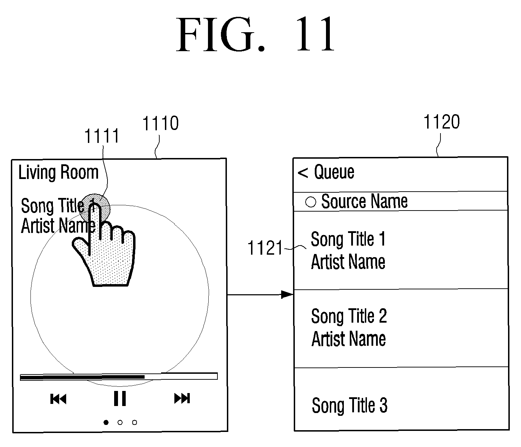

Referring to FIG. 11, a screen 1110 for controlling an audio content (Song Title 1) which is being outputted through a speaker at a living room is displayed. In response to a user's touch 1111 with respect to the audio content (Song Title 1) being detected in this state, the processor 130 may display an audio content list 1120 including a plurality of audio contents. In this case, the audio content (Song Title 1) which is currently being outputted may be displayed in a color different from a color of other audio contents (Song Title 2 and Song Title 3) in the audio content list.

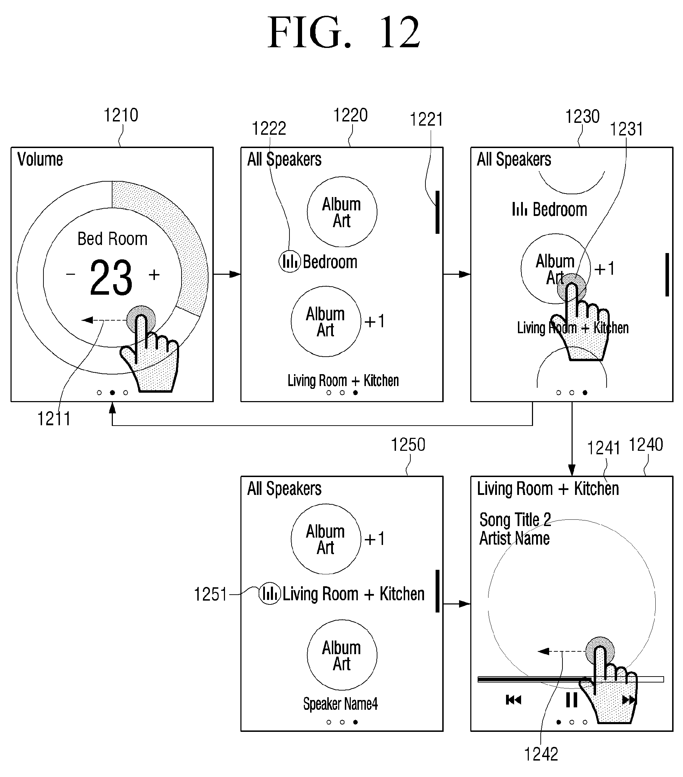

FIG. 12 illustrates a process of changing a speaker for outputting an audio content according to an exemplary embodiment.

Referring to FIG. 12, in response to a user's manipulation 1211 of dragging a pre-executed user's touch being detected while a screen 1210 for adjusting a volume level of a speaker at a bedroom is displayed, the processor 130 may display a speaker list 1220 including entire speakers connected to an external apparatus.

In this case, the speaker list 1220 may include an equalizer-shaped icon 1222 representing that a speaker which is reproducing an audio content is the speaker at the bedroom. Not all of the speakers connected with the external apparatus may be displayed on the screen. In this case, the speaker list 1220 may display a scroll bar 1221 such that a user may scroll up or down to view all of the speakers connected with the external apparatus.

In response to a user's touch 1231 with respect to an icon corresponding to a group including a speaker at a living room and a speaker at a kitchen being detected while the speaker list 1220 is scrolled down and a screen 1230 is displayed, the processor 130 may transmit a control command for interrupting reproduction of an audio content through the speaker at the bedroom and reproducing the audio content through the speakers at the living room and the kitchen to an external apparatus.

In response to a user's manipulation of dragging a pre-executed user's touch to the right being detected while the speaker list 1220 is displayed, the processor 130 may display the screen 1210 for adjusting the volume level of the speaker at the bedroom.

In addition, the processor 130 may display a screen 1240 for controlling an audio content (Song Title 2) which is currently being outputted through the speaker at the living room and the speaker at the kitchen. In this case, the screen 1240 for controlling the audio content (Song Title 2) which is currently being reproduced through the speaker at the living room and the speaker at the kitchen may include text 1241 representing that speakers which are reproducing the audio content are the speaker at the living room and the speaker at the kitchen.

In response to a user's manipulation 1242 of dragging a pre-executed user's touch to the left being detected while the screen 1240 for controlling the audio content (Song Title 2) which is currently being outputted through the speaker at the living room and the speaker at the kitchen is displayed, the processor 130 may display a speaker list 1250 including entire speakers connected with the external apparatus with a scroll bar. In this case, the speaker list 1250 may include an equalizer-shaped icon 1251 representing that the speakers which are reproducing the audio content are the speaker at the living room and the speaker at the kitchen.

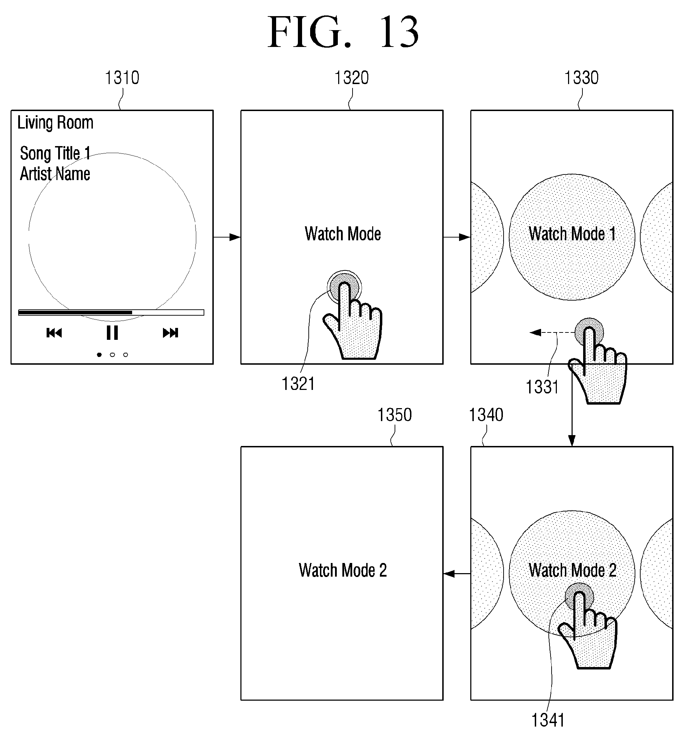

FIG. 13 illustrates UI screens related to a clock mode according to an exemplary embodiment.

Referring to FIG. 13, in response to no user's manipulation being detected within a predetermined time while a screen 1310 for controlling an audio content which is being outputted through a speaker at a living room is displayed, the processor 130 may operate in a clock mode (or watch mode) and display a clock screen 1320 which displays a current time to correspond to the clock mode.

In response to a user's touch 1321 with respect to the clock screen 1320 being detected while the clock screen 1320 is displayed, the processor 130 may display a screen 1330 for selecting a first clock screen corresponding to a first clock mode. In response to a user's manipulation 1331 of dragging a pre-executed user's touch to the left being detected, the processor 130 may display a screen 1340 for selecting a second clock screen corresponding to a second clock mode.

In addition, in response to a user's touch 1341 with respect to the second clock screen being detected while the screen 1340 for selecting the second clock screen corresponding to the second clock mode is displayed, the processor 130 may display a second clock screen 1350 selected by the user's touch 1341.

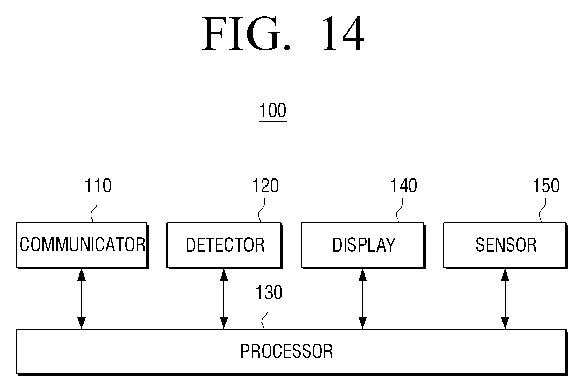

FIG. 14 is a block diagram illustrating a structure of a user terminal apparatus according to another exemplary embodiment.

Referring to FIG. 14, a user terminal apparatus 100 includes a communicator 110, a detector 120, a processor 130, a display 140, and a sensor 150. The communicator 110, the detector 120, the processor 130, and the display 140 have been described above, and thus, a detailed description thereof will be omitted.

The sensor 150 may sense location information of the user terminal apparatus 100. In an exemplary embodiment, the sensor 150 may sense the location information of the user terminal apparatus 100 by using a global positioning system (GPS) sensor.

In addition, in response to determining that at least one speaker among a plurality of speakers exists within a predetermined distance range from the user terminal apparatus 100 based on the location information on the user terminal apparatus 100, the processor 130 may display a UI screen for automatically controlling the speaker which exists within the predetermined distance range.

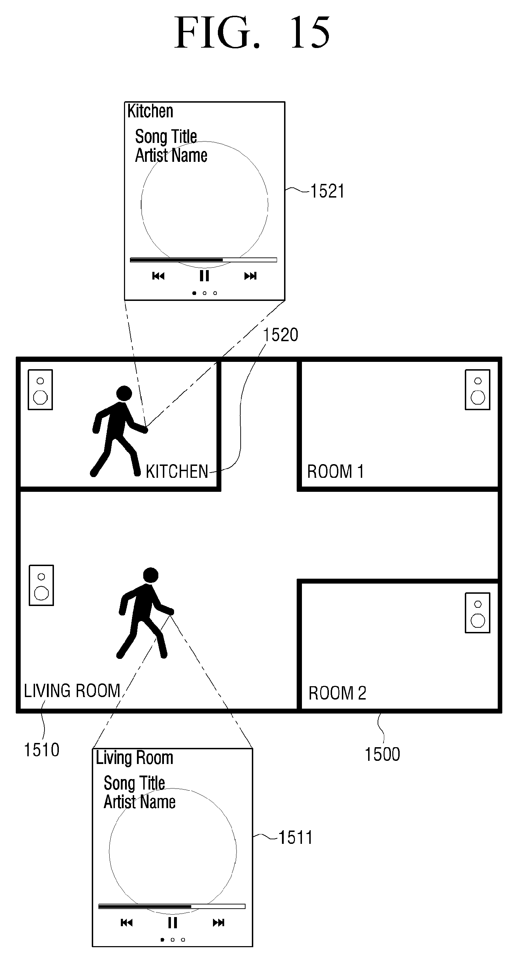

FIG. 15 is a view illustrating a speaker control screen displayed based on location information of a user terminal apparatus according to an exemplary embodiment.

Referring to FIG. 15, in response to detecting that a user is at a living room 1510 in a home 1500, the processor 130 may display a UI screen 1511 for controlling a speaker located at the living room which exists within a predetermined distance range from a user terminal apparatus 100 among a plurality of speakers connected with an external apparatus based on location information of the user terminal apparatus 100.

In response to the user moving from the living room 1510 to a kitchen 1520, the processor 130 may display a UI screen 1521 for controlling a speaker located at the kitchen within a predetermined distance range from the user terminal apparatus 100 among the plurality of speakers connected with the external apparatus based on the location information of the user terminal apparatus 100.

That is, the processor 130 may specify a speaker within a predetermined distance from the user terminal apparatus 100 from among the plurality of speakers connected with the external apparatus based on the location information of the user terminal apparatus 100 and display a UI screen for controlling the specified speaker. The UI screen for controlling the specified speaker may be displayed automatically or upon a request.

Accordingly, the user may control the specified speaker by performing a user's manipulation with respect to a UI screen for controlling the specified speaker which is displayed automatically or upon a request, without searching for a desired speaker from among the plurality of speakers.

In addition, the above-described various user's manipulations (e.g., a user's touch of covering a UI screen, a predetermined user's motion being recognized within a predetermined distance from a UI screen, and the like) may be applied in the same manner to the UI screen 1511 for controlling the speaker located at the living room which exists within the predetermined distance from the user terminal apparatus 100 among the plurality of speakers connected with the external apparatus based on the location information of the user terminal apparatus 100.

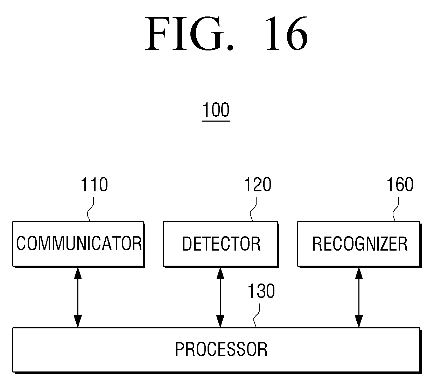

FIG. 16 is a block diagram illustrating a structure of a user terminal apparatus according to still another exemplary embodiment.

Referring to FIG. 16, a user terminal apparatus 100 may include a communicator 110, a detector 120, a processor 130, and a recognizer 160. The communicator 110, the detector 120, and the processor 130 have been described above, and thus, a detailed description thereof will be omitted.

The recognizer 160 may recognize a user's uttered voice. In an exemplary embodiment, the recognizer 160 may generate text corresponding to the user's uttered voice by converting the user's uttered voice into text through an automatic speech recognition (ASR) module. The ASR module refers to a module for converting a voice signal into text. Accordingly, a voice signal may be converted into text by using a variety of conventional ASR algorithms.

For example, the processor 130 detects a start point and an end point of a user's uttered voice from a received voice signal to determine a voice section. In an exemplary embodiment, the processor 130 may calculate energy of the received voice signal, divide an energy level of the voice signal according to the calculated energy, and detect a voice section through dynamic programming. Subsequently, the processor 130 may detect a phoneme, that is, the smallest unit of a voice, from the detected voice section based on an acoustic model, generate phonemic data, and apply a hidden Markov model (HMM) probabilistic model to convert the user's uttered voice into text.

In addition, the processor 130 may perform diverse analyses, such as, part of speech, named entity extraction, information extraction, semantic analytic, and the like, with respect to the text corresponding to the user's uttered voice by using a spoken language understanding (SLU) module such that the meaning of the text is determined by the processor 130.

Subsequently, the processor 130 may detect a corpus database including a conversation pattern matching the text converted from the user's uttered voice, detect a domain corresponding to the user's uttered voice, and recognize the user's uttered voice from the detected domain.

In response to a user's uttered voice for controlling at least one of a plurality of speakers being recognized, the processor 130 may transmit a control command corresponding to the user's uttered voice to the external apparatus.

In response to the user's uttered voice being recognized, the processor 130 may retrieve an audio content based on the recognized user's uttered voice. For example, the processor 130 may retrieve an audio content by using a name of the audio content, an album title including the audio content, and an artist's name related to the audio content based on the user's uttered voice.

In addition, the processor 130 may transmit a control command for outputting the retrieved audio content through a speaker specified by the user's uttered voice to the external apparatus.

As an example, in response to a user's uttered voice "Play music entitled 'OOO' through a speaker at a living room" being recognized, the processor 130 may transmit a control command for retrieving the music 'OOO' and outputting the retrieved music through the speaker at the living room to the external apparatus.

As another example, in response to a user's uttered voice "Output a sound of a TV through a speaker at a living room" being recognized, the processor 130 may transmit a control command for outputting the sound of the TV through the speaker at the living room to the external apparatus.

However, exemplary embodiments are not limited to this and, in response to determining that the speaker at the living room exists within a predetermined distance range from the user terminal apparatus based on the location information of the user terminal apparatus 100 and a user's uttered voice "Play music entitled 'OOO'" being recognized, the processor 130 may transmit a control command for automatically outputting the music 'OOO' through the speaker at the living room to the external apparatus.

The sensor 150 may sense time information and biometric information. In this case, the time information refers to a current time and may be displayed on a display of the user terminal apparatus 100. The time information may be sensed by using a GPS sensor. The biometric information refers to an electrical signal of micro cells of a human body and includes a brainwave, an electromyogram (EMG), an electrocardiogram (ECG), and the like, for example. In addition, the sensor 150 may sense a heart rate, a breathing rate, a moving distance, the number of steps, a duration of an action, the number of times of moving a finger, a voice volume, the number of times of using a particular word pattern, a duration of an emotional state, the number of times of changes of an emotional state, and the like, as the biometric information.

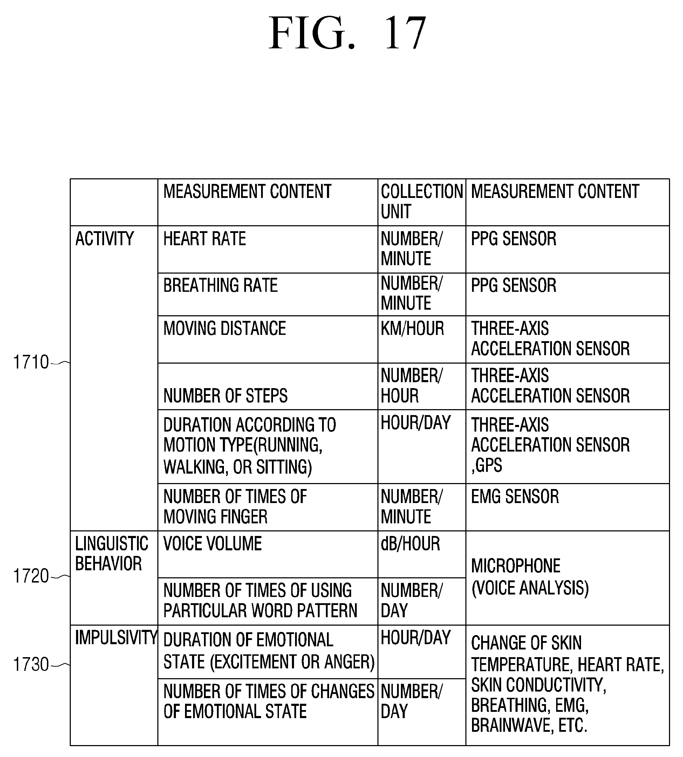

FIG. 17 is a view illustrating biometric information according to an exemplary embodiment.

Referring to FIG. 17, the biometric information may be divided into, for example, an activity category 1710, a linguistic behavior category 1720, and an impulsivity category 1730. The activity category 1710 may include information on a heart rate, a breathing rate, a moving distance, the number of steps, a duration of a motion of a particular type (e.g., running, walking, or sitting), and the number of moving a finger. The linguistic behavior category 1720 may include information on a voice volume and the number of times of using a particular word pattern. The impulsivity category 1730 may include information on a duration of an emotional state (e.g., excitement or anger) and the number of times of changes of an emotional state. However, exemplary embodiments are not limited to this and, the biometric information is not limited to the above-described examples of FIG. 17 and may include any information on a user's bio-signal, voice, motion, emotional change, movement, and the like, relevant to a wearable user terminal apparatus.

The sensor 150 may sense the biometric information on a user on a predetermined cycle. For example, the sensor 150 may include a photoplethysmographic (PPG) sensor. The PPG sensor included in the sensor 150 may sense the number of times a user's heart beats in one minute and the number of times the user breaths in one minute. An acceleration sensor included in the sensor 150 may sense a user's moving distance, that is, the distance (e.g., several kilometers) the user moves in one hour, sense the number of steps the user walks in one hour, and sense the duration of a user's action, for example, the running, walking, and sitting, in a day. In addition, the sensor 150 may sense an average decibel (dB) of a volume of a user's voice per day and sense the number of times a particular word pattern is used in a day.

In addition, the sensor 150 may include a galvanic skin response (GSR) sensor. The GSR sensor included in the sensor 150 may sense the duration of a user's emotional state (e.g., excitement or anger) in a day and/or the number of times the emotional state is changed in a day.

The processor 130 may transmit a control command for specifying at least one of a plurality of speakers based on the location of the user terminal apparatus and retrieving an audio content based on at least one of the time information and the biometric information to the external apparatus along with the location information of the user terminal apparatus, time information, and biometric information.

For example, the biometric information, such as, the heart rate, the breathing rate, and the duration of an emotional state may be sensed by the sensor 150. In this case, in response to determining that the sensed heart rate and/or breathing rate are higher than a predetermined average value and/or a user's excited state lasts over a predetermined time based on the sensed heart rate, breathing rate, and the duration of the emotional state, the processor 130 may transmit a control command for retrieving an audio content that has at a quick tempo and a vibrant feel to the external apparatus.

In addition, the processor 130 may transmit a control command for retrieving an audio content based on the time information and biometric information sensed by the sensor 150, to the external apparatus.

That is, the processor 130 may generate a control command for retrieving an audio content based on the sensed time information and biometric information, specifying a speaker based on the location information of the user terminal apparatus 100, and outputting the audio content through the specified speaker and transmit the generated control command to the external apparatus without a user's manipulation of selecting an audio content and specifying a speaker for outputting the selected audio content.

For example, in response to the sensed time information indicating two o'clock in the afternoon and the sensed heart rate and/or the breathing rate being higher than a predetermined average value, the processor 130 may transmit a control command for retrieving an audio content related to rock music or pop music from among diverse music genres to the external apparatus.

In response to the sensed time information indicating eight o'clock in the evening and the sensed heart rate and/or the breathing rate being higher than the predetermined average value, the processor 130 may transmit a control command for retrieving an audio content related to jazz music from among diverse music genres to the external apparatus.

In response to the sensed biometric information relating to a brainwave and determining that the sensed brainwave represents a user's sleeping state, the processor 130 may generate a control command for turning off a speaker which is currently outputting an audio content and transmit the generated control command to the external apparatus.

In response to the sensed biometric information relating to a brainwave and determining that the sensed brainwave represents a user's awakening state switched from the sleeping state, the processor 130 may generate a control command for retrieving and outputting an alarm sound or an audio content of a genre in a vibrant mood (e.g., rock music, metal music, pop music, and the like) and transmit the generated control command to the external apparatus.

In response to the sensed time information indicating eight o'clock in the morning and determining that the sensed brainwave represents an awakening state, the processor 130 may transmit a control command for retrieving and outputting an audio content in a vibrant mood to the external apparatus based on the time information represents the morning time. In response to the sensed time information indicating eleven o'clock in the evening and determining that the sensed brainwave represents an awakening state, the processor 130 may transmit a control command for retrieving and outputting an audio content in a calming mood to the external apparatus based on the time information that represents the night time.

The processor 130 may specify at least one of a plurality of speaker based on the location information of the user terminal apparatus. For example, in response to a user sitting on a sofa at a living room and wearing a user terminal apparatus, the processor 130 may recognize that a speaker at the living room is located within a predetermined distance range from the user terminal apparatus based on the location information of the user terminal apparatus 100. Subsequently, the processor 130 may transmit a control command for retrieving an audio content based on the sensed time information and biometric information and outputting the retrieved audio content through the speaker at the living room to the external apparatus, as described above.

For example, in response to determining that the speaker which is located within the predetermined distance range from the user terminal apparatus is the speaker at the living room based on the location information of the user terminal apparatus 100, the sensed time information indicates two o'clock in the afternoon, and the sensed heart rate and/or the breathing rate are higher than a predetermined average value, the processor 130 may transmit a control command for retrieving an audio content related to the rock music or pop music from among diverse music genres and outputting the retrieved audio content through the speaker at the living room to the external apparatus.

The processor 130 may transmit the location information of the user terminal apparatus 100, the time information, and the biometric information to the external apparatus along with a control command such that the external apparatus may retrieve an audio content based on the received information. Accordingly, the external apparatus may retrieve an audio content based on the received location information of the user terminal apparatus 100, time information, biometric information, and the control command and output the retrieved audio content through a speaker specified based on the location information of the user terminal apparatus 100.

In an exemplary embodiment, in response to determining that a distance between the user terminal apparatus and the external apparatus exceeds a predetermined threshold value based on the location information of the user terminal apparatus, the processor 130 may transmit a control command for turning off a plurality of speakers connected with the external apparatus to the external apparatus.

For example, in response to determining that the distance between the user terminal apparatus 100 and the external apparatus is out of a coverage range for Bluetooth communication, the processor 130 may transmit a control command for turning off the plurality of speakers connected with the external apparatus to the external apparatus.

Accordingly, in response to the distance between the user terminal apparatus 100 and the external apparatus being determined as a predetermined threshold value or more, the user may control to turn off the plurality of speakers by transmitting a control command for turning off the plurality of speakers at home to the external apparatus without individually turning off the plurality of speakers at home when the user leaves home. For example, the user may control to turn off the plurality of speakers automatically or upon a request.

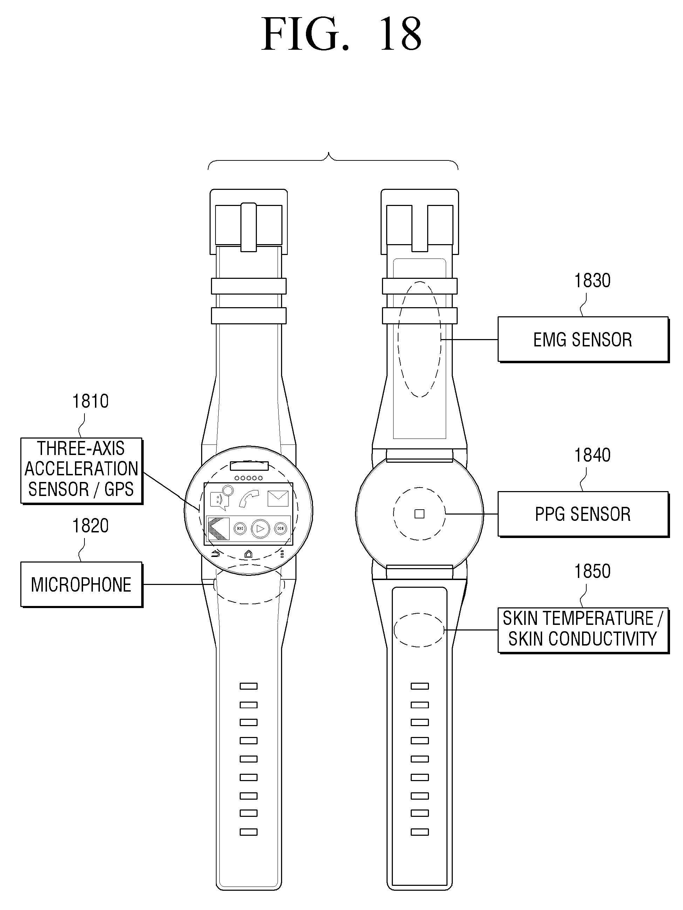

FIG. 18 is a view illustrating a user terminal apparatus including various sensors according to an exemplary embodiment.

Referring to FIG. 18, the user terminal apparatus 100 is provided as a smart watch. A three-axis acceleration sensor or a GPS sensor 1810 may be embedded in one surface, e.g., a front surface of a smart watch, and a microphone 1820 may be mounted on or adjacent to one surface of a display of the smart watch. An EMG sensor 1830 and a skin temperature/skin conductivity sensor 1850 may be mounted on, for example, a band of the smart watch. In addition, a PPG sensor 1840 may be embedded in one surface, e.g., a rear surface of the display of the smart watch. The array of the above-described various sensors illustrated in FIG. 18 is only an example and exemplary embodiments are not limited to this. For example, and the sensors may be arrayed at various positions in the smart watch and the band.

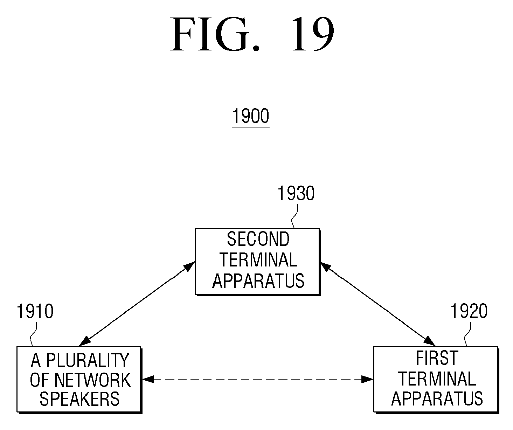

FIG. 19 is a block diagram illustrating a structure of a system according to an exemplary embodiment.

Referring to FIG. 19, a system 1900 may include a plurality of network speakers 1910, a first terminal apparatus 1920, and a second terminal apparatus 1930. In this case, the plurality of network speakers 1910, the first terminal apparatus 1920, and the second terminal apparatus 1930 may respectively correspond to the plurality of speakers, the user terminal apparatus 100, and the external apparatus as described in the above-described exemplary embodiments.

In an exemplary embodiment, the second terminal apparatus 1930 may directly or indirectly control the plurality of network speakers 1910. In addition, the second terminal apparatus 1930 may be connected to the same access point and form the same network with the plurality of network speakers 1910.

The first terminal apparatus 1920 may transmit a control command for controlling the plurality of network speakers 1910 to the second terminal apparatus 1930 such that the second terminal apparatus 1930 may directly or indirectly control the plurality of network speakers 1910.

For example, the first terminal apparatus 1920 may transmit a control command for controlling the plurality of network speakers 1910 to the second terminal apparatus 1930, and in response to the control command being received, the second terminal apparatus 1930 may control the plurality of network speakers 1910 according to the received control command.

In this case, the first terminal apparatus 1920 may detect a user's touch on the first terminal apparatus 1920 and generate a control command for controlling the plurality of network speakers 1910 according to a type of the user's touch.

In an exemplary embodiment, the first terminal apparatus 1920 may display a UI screen for controlling at least one of the plurality of speakers. In response to a user's touch of covering the entire UI screen displayed on the first terminal apparatus 1920 being detected, the first terminal apparatus 1920 may generate a control command for muting at least one of the plurality of speakers and transmit the generated control command to the second terminal apparatus 1930.

As an example, a UI screen for controlling a speaker at a living room and a speaker at a kitchen among the plurality of network speakers 1910 is displayed, and in response to a user's touch of covering the entire UI screen being detected, the first terminal apparatus 1920 may generate a control command for muting the speakers at the living room and the kitchen and transmit the generated control command to the second terminal apparatus 1930. That is, a speaker which is not at the living room and kitchen among the plurality of speakers, for example, a speaker at a bedroom or a speaker at a bathroom, is not to be muted according to the control command.

In addition, as another example, a UI screen for controlling all of the plurality of network speakers 1910 is displayed, and in response to a user's touch of covering the entire UI screen being detected, the first terminal apparatus 1920 may generate a control command for muting all of the plurality of network speakers 1910 and transmit the generated control command to the second terminal apparatus 1930.

In addition, in response to a predetermined user's motion being detected within a predetermined distance from a UI screen, the first terminal apparatus 1920 may generate a control command for decreasing a volume level of at least one of the plurality of network speakers and transmit the generated control command to the second terminal apparatus 1930.

For example, in response to a user's motion of moving a hand to a UI screen within a predetermined distance from the UI screen being detected while the UI screen for controlling an audio content is displayed, the first terminal apparatus 1920 may generate a control command for decreasing a volume level of a speaker of which the UI screen is displayed for controlling the audio content and transmit the generated control command to the second terminal apparatus 1930.

As an example, a UI screen indicating that a speaker at a kitchen is reproducing an audio content is displayed, and in response to a user's motion of moving a hand to the UI screen within a range of five centimeter from the UI screen being detected, the first terminal apparatus 1920 may generate a control command for decreasing a volume level of the speaker at the kitchen and transmit the generated control command to the second terminal apparatus 1930.

As another example, a UI screen indicating that the speaker at the kitchen and a speaker at a living room are grouped into one group and reproducing an audio content is displayed, and in response to a user's motion of moving a hand to the UI screen within a range of, for example, five centimeters from the UI screen being detected, the first terminal apparatus 1920 may generate a control command for decreasing a volume level of the speakers at the kitchen and the living room and transmit the generated control command to the second terminal apparatus 1930.

In addition, the first terminal apparatus 1920 may generate a control command for decreasing a volume level of a speaker of which UI screen is displayed and transmit the generated control command to the second terminal apparatus 1930 based on a speed at which the hand approaches the UI screen within the predetermined distance from the UI screen and based on a distance between the UI screen and the hand.

For example, a UI screen indicating that the speaker at the kitchen is reproducing an audio content is displayed, and in response to a speed at which the hand approaches the UI screen within a range of, for example, five centimeters from the UI screen being increased, the first terminal apparatus 1920 may generate a control command for decreasing a volume level of the speaker at the kitchen and transmit the generated control command to the second terminal apparatus 1930. In addition, when the hand approaches the UI screen and the distance between the hand and the UI screen decreases, the first terminal apparatus 1920 may generate a control command for increasing a speed of decreasing the volume level of the speaker at the kitchen according to the decreased distance between the hand and the UI and transmit the generated control command to the second terminal apparatus 1930.

The above-described operations of the user terminal apparatus 100 may be applied in the same or similar manner to the first terminal apparatus 1920 of FIG. 19, and the second terminal apparatus 1930 may operate in the same or similar manner as operations of the external apparatus described above.

The first terminal apparatus 1920 may transmit a control command for controlling the plurality of network speakers 1910 to the second terminal apparatus 1930, and the second terminal apparatus 1930 may control the plurality of network speakers 1910 according to the received control command. In an exemplary embodiment, the first terminal apparatus 1920 may directly or indirectly communicate with the plurality of network speakers 1910 to control the plurality of network speakers 1910 without the second terminal apparatus 1930.

In this case, the first terminal apparatus 1920 may directly or indirectly transmit the control command for controlling the plurality of network speakers 1910 to the plurality of network speakers 1910 and thus control the plurality of network speakers 1910 without transmitting the control command to the second terminal apparatus 1930. In this case, the control command transmitted from the first terminal apparatus 1920 to the plurality of network speakers 1910 may be the same as the control command transmitted from the user terminal apparatus 100 to the external apparatus.

It is assumed that the first terminal apparatus 1920 of FIG. 19 is a TV, and the second terminal apparatus 1930 is a smart phone. In this case, the TV may display a speaker list including the plurality of network speakers 1910 connected with the smart phone and adjust a volume level of the plurality of network speakers.

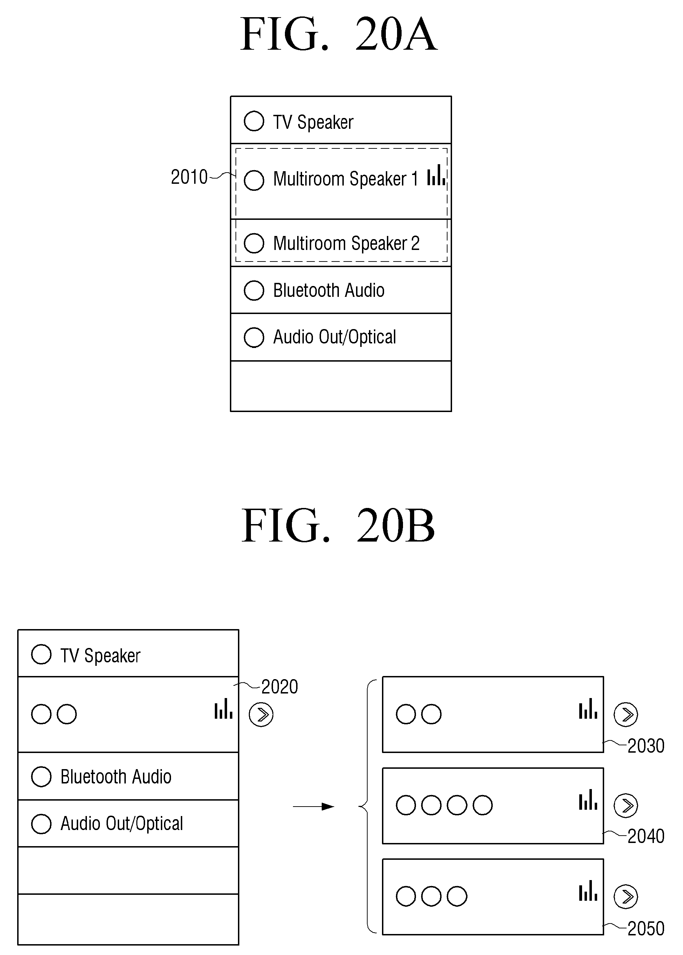

FIGS. 20A and 20B are views illustrating a process of adjusting a volume level of a plurality of network speakers in a TV according to an exemplary embodiment.

Referring to FIG. 20A, a TV may display a speaker list including the plurality of network speakers 1910 connected with the smart phone. In response to a user's touch with respect to speakers 2010 (e.g., 'Multiroom Speaker 1' and 'Multiroom Speaker 2') included in the displayed speaker list, the TV may display information on the touched speakers 2010. For example, the TV may display information on a location, an output amount, a model number, and a channel of the speakers 2010.

In addition, in response to a user interaction with respect to the speakers 2010 the TV may individually generate control commands with respect to the speakers 2010 among the plurality of network speakers 1910 and transmit the generated control commands to the smart phone.

Referring to FIG. 20B, in response to a user's manipulation of grouping the speakers 2010 among the plurality of network speakers 1910, the TV may group and display the speakers 2010 among the plurality of network speakers 1910 into one group.

In this case, in response to two speakers among the plurality of network speakers 1910 being grouped into surround two-channel speakers, the TV may group the two speakers and display icons corresponding to the two speakers that are grouped into one group 2030. In response to four speakers among the plurality of network speakers 1910 being grouped into surround four-channel speakers, the TV may group the four speakers and display icons corresponding to the four speakers that are grouped into one group 2040.

In addition, in response to three speakers among the plurality of network speakers 1910 being grouped into a combination of a sound bar speaker and the surround two-channel speakers, the TV may group the three speakers and display icons corresponding to the three speakers that are grouped into one group 2050.

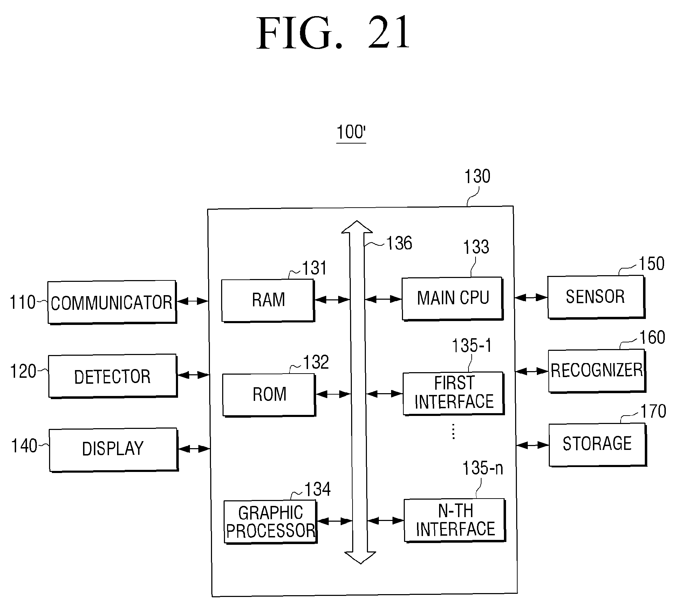

FIG. 21 is a block diagram illustrating a detailed structure of the user terminal apparatus of FIG. 1.

Referring to FIG. 21, a user terminal apparatus 100' includes a communicator 110, a detector 120, a processor 130, a display 140, a sensor 150, a recognizer 160 , and a storage 170. A repetitive description on the components illustrated in FIGS. 1, 2, 14, and 16 will be omitted.

The processor 130 controls overall operations of the user terminal apparatus 100'.

In an exemplary embodiment, the processor 130 includes a random access memory (RAM) 131, a read-only memory (ROM) 132, a main central processing unit (CPU) 133, a graphic processor 134, a first to n-th interfaces 135-1 to 135-n, and a bus 136.

The RAM 131, the ROM 132, the main CPU 133, the graphic processor 134, and the first to n-th interfaces 135-1 to 135-n may be interconnected to each other through the bus 136.

The first to n-th interfaces 135-1 to 135-n may be connected to the aforementioned various components. One of the first to n-th interfaces 135-1 to 135-n may be a network interface which is connected to an external apparatus through a network.

The main CPU 133 accesses the storage 150 and performs a boot-up operation by using an operating system (O/S) stored in the storage 170. In addition, the main CPU 133 performs various operations by using diverse programs, contents, and data stored in the storage 170.

The ROM 132 stores a set of commands for system booting. In response to a turn-on command being received and power being supplied, the main CPU 133 copies the O/S stored in the storage 170 to the RAM 131 according to a command stored in the ROM 132, and boots up a system by executing the O/S. Upon completion of the boot-up operation, the main CPU 133 copies various application programs stored in the storage 170 to the RAM 131 and executes the application programs copied to the RAM 131 to perform various operations.

The graphic processor 134 generates a screen including various objects, such as, an icon, an image, text, etc., by using a computing unit (not shown) and a rendering unit (not shown). The computing unit (not shown) computes attribute values, such as, a coordinate value, a shape, a size, and a color of each object to be displayed, according to a layout of the screen based on the received control command. The rendering unit (not shown) generates a screen with various layouts including objects based on the attribute values computed by the computing unit. For example, the graphic processor 134 may convert a system response generated in response to a user's uttered voice into text and determine a font, size, and color of a letter of the text. The screen generated by the rendering unit (not shown) may be displayed on a display area of the display 140.

The above-described operations of the processor 130 may be performed by the programs stored in the storage 170.

The storage 170 stores various data, such as, an O/S software module for operating the user terminal apparatus 100' and diverse multimedia contents.

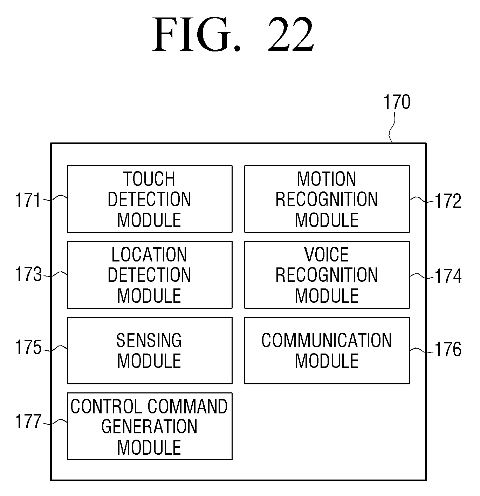

For example, the storage 170 includes a software module for generating a control command for controlling a plurality of speakers according to a type of a user's touch and transmitting the generated control command to an external application. This operation will be described below in further detail with reference to FIG. 22.

FIG. 22 is a view illustrating software modules stored in a storage according to an exemplary embodiment.

Referring to FIG. 22, a storage 170 may include programs, such as, a touch detection module 171, a motion recognition module 172, a location detection module 173, a voice recognition module 174, a sensing module 175, a communication module 176, and a control command generation module 177.

The above-described operations of the processor 130 may be performed by using the programs stored in the storage 170. Hereinafter, the processor's operations performed by using the programs stored in the storage 170 will be described in detail.

The touch detection module 171 may detect a user's touch. For example, the touch detection module 171 may detect a user's touch and calculate a coordinate of a touch point.

The motion recognition module 172 may recognize a user's motion by comparing a detected user's motion with a predetermined motion.

The location detection module 173 may detect a current location of the user terminal apparatus 100 based on location information of the user terminal apparatus 100.

The voice recognition module 174 may generate text corresponding to a user's uttered voice by converting the user's uttered voice into text and recognize the user's uttered voice by analyzing the generated text.

The sensing module 175 collects information from various sensors and analyzes and manages the collected information. In an exemplary embodiment, the sensing module 175 may sense the location information of the user terminal apparatus or sense time information and biometric information.

The communication module 176 performs communication or pairing with an external apparatus. The communication module 176 may include a device module used for communication with an external apparatus, a messaging module including a messenger program, a short message service (SMS) and multimedia message service (MMS) program, and an e-mail program, a call info aggregator program module, and a phone module including a voice over Internet protocol (VoIP) module.

The control command generation module 177 may generate a control command for controlling a plurality of network speakers based on at least one of a type of a user's touch, the location information of the user terminal apparatus 100, the time information, and the biometric information.

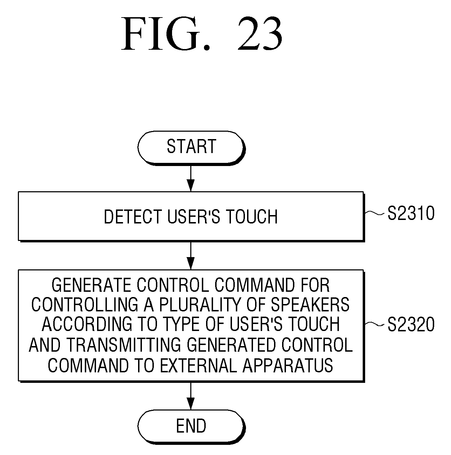

FIG. 23 is a flowchart provided to describe a method for controlling a user terminal apparatus according to an exemplary embodiment.

According to a method for controlling a user terminal apparatus of FIG. 23, a user's touch is detected in operation S2310.

A control command for controlling a plurality of speakers is generated according to a type of the user's touch and transmitted to an external apparatus in operation S2320.

The method may further include displaying a UI screen for controlling at least one of the plurality of speakers. In response to a user's touch of covering the entire UI screen being detected, the transmitting in operation S2320 may include generating a control command for muting at least one of the plurality of speakers and transmitting the generated control command to the external apparatus.

In response to a predetermined user's motion being detected within a predetermined distance from the UI screen, the transmitting in operation S2320 may include generating a control command for decreasing a volume level of at least one of the plurality of speakers and transmitting the generated control command to the external apparatus.

In addition, in response to a user's manipulation of controlling at least one of the plurality of speakers being received with respect to the UI screen, the transmitting in operation S2320 may include transmitting a control command corresponding to the user's manipulation to the external apparatus.

The method for controlling the user terminal apparatus according to an exemplary embodiment may further include sensing location information of the user terminal apparatus. In addition, in response to determining that at least one speaker among the plurality of speakers is located within a predetermined distance range from the user terminal apparatus based on the location information of the user terminal apparatus, the method may further include automatically displaying a UI screen for controlling the speaker which is located within the predetermined distance range.

The method for controlling the user terminal apparatus according to an exemplary embodiment may further include recognizing a user's uttered voice. In response to the user's uttered voice for controlling at least one of the plurality of speakers being recognized, the method may further include transmitting a control command corresponding to the recognized user's uttered voice to the external apparatus.

In response to the user's uttered voice being recognized, the method may further include transmitting a control command for retrieving an audio content based on the recognized user's uttered voice and outputting the audio content through a speaker specified by the user's uttered voice to the external apparatus.

In addition, the method for controlling the user terminal apparatus according to an embodiment may further include sensing time information and biometric information. The method may further include transmitting a control command for specifying at least one of the plurality of speakers based on the location information of the user terminal apparatus and retrieving an audio content based on at least one of the time information and biometric information to the external apparatus along with the location information of the user terminal apparatus, time information, and biometric information.

The method for controlling a first electronic apparatus according to the above-described various exemplary embodiments may be embodied as a program code which is executable by a computer, stored in diverse non-transitory computer readable mediums, and provided to each apparatus so as to be executed by a controller.

As an example, a non-transitory computer readable medium in which a program, which, when executed by a computer, causes the computer to execute a control method including detecting a user's touch, generating a control command for controlling a plurality of speakers according to a type of the user's touch, and transmitting the generated control command to an external apparatus is stored may be provided.

The non-transitory computer readable medium refers to a medium which may store data permanently or semi-permanently rather than storing data for a short time, such as, a register, a cache, a memory, and the like, and may be readable by an apparatus. In an exemplary embodiment, the above-described various applications and programs may be stored in and provided through the non-transitory computer readable medium, such as, a compact disc (CD), a digital versatile disk (DVD), a hard disk, a Blu-ray disk, a universal serial bus (USB), a memory card, a read-only memory (ROM), etc.

According to the above-described various exemplary embodiments, a plurality of speakers may be controlled through various user's manipulations with respect to a user terminal apparatus.

At least one of the components, elements or units represented by a block as illustrated in the drawings may be embodied as various numbers of hardware, software and/or firmware structures that execute respective functions described above, according to an exemplary embodiment. For example, at least one of these components, elements or units may use a direct circuit structure, such as a memory, processing, logic, a look-up table, etc. that may execute the respective functions through controls of one or more microprocessors or other control apparatuses. Also, at least one of these components, elements or units may be specifically embodied by a module, a program, or a part of code, which contains one or more executable instructions for performing specified logic functions. Also, at least one of these components, elements or units may further include a processor such as a central processing unit (CPU) that performs the respective functions, a microprocessor, or the like. Further, although a bus is not illustrated in some of the block diagrams, communication between the components, elements or units may be performed through the bus. Functional aspects of the above exemplary embodiments may be implemented in algorithms that execute on one or more processors. Furthermore, the components, elements or units represented by a block or processing steps may employ any number of related art techniques for electronics configuration, signal processing and/or control, data processing and the like.

Although a few embodiments have been shown and described, it would be appreciated by those skilled in the art that changes may be made in the exemplary embodiments without departing from the principles and spirit of the disclosure, the scope of which is defined in the claims and their equivalents.

## Claims

1. A user terminal apparatus comprising:
a communicator configured to communicate with an external apparatus;
a detector configured to detect a touch operation of a user with respect to the user terminal apparatus; and
a processor configured to control the communicator to generate a control command for controlling at least one of a plurality of speakers according to a type of the touch operation and configured to transmit the generated control command to the external apparatus.

2. The apparatus as claimed in claim 1, further comprising:
a display configured to display a user interface (UI) screen associated with the at least one of the plurality of speakers,
wherein, in response to a touch operation in which a substantial portion of the UI screen is covered, the processor is configured to generate a control command for muting the at least one of the plurality of speakers and configured to transmit the generated control command to the external apparatus.

3. The apparatus as claimed in claim 2, wherein in response to a predetermined motion of the user being recognized within a predetermined distance from the UI screen, the processor is configured to generate a control command for decreasing a volume level of the at least one of the plurality of speakers and transmit the generated control command to the external apparatus.

4. The apparatus as claimed in claim 2 or 3, wherein in response to a user manipulation for controlling the at least one of the plurality of speakers being received with respect to the UI screen, the processor is configured to transmit a control command corresponding to the user manipulation to the external apparatus.

5. The apparatus as claimed in any one of claims 2 to 4, further comprising:
a sensor configured to sense location information of the user terminal apparatus,
wherein in response to determining that at least one speaker among the plurality of speakers exists within a predetermined distance from the user terminal apparatus based on the location information of the user terminal apparatus, the processor is configured to automatically display a UI screen for controlling the at least one speaker.

6. The apparatus as claimed in any one of claims 1 to 5, further comprising:
a recognizer configured to recognize a voice of the user,
wherein in response to a voice of the user for controlling the at least one of the plurality of speakers being recognized, the processor is configured to transmit, to the external apparatus, a control command for controlling the at least one of the plurality of speakers based on the recognized voice.

7. The apparatus as claimed in claim 6, wherein in response to the voice of the user for controlling the at least one of the plurality of speakers being recognized, the processer is configured to transmit, to the external apparatus, a control command for retrieving, based on the recognized voice, an audio content and outputting the audio content through a speaker specified by the voice.

8. The apparatus as claimed in any one of claims 5 to 7, wherein the sensor is configured to sense at least one of time information and biometric information,
wherein the processor is configured to transmit, to the external apparatus, a control command for specifying, based on the location information on the user terminal apparatus, the at least one of the plurality of speakers and retrieving, based on the at least one of the time information and the biometric information, an audio content.

9. The apparatus as claimed in any one of claims 5 to 8, wherein in response to determining that a distance between the user terminal apparatus and the external apparatus exceeds a predetermined threshold value based on the location information on the user terminal apparatus, the processor is configured to transmit a control command for turning off the plurality of speakers connected with the external apparatus, to the external apparatus.

10. A method for controlling a user terminal apparatus which communicates with an external apparatus that controls a plurality of speakers, the method comprising:
detecting a touch operation of a user; and
generating a control command for controlling at least one of the plurality of speakers according to a type of the touch operation and transmitting the generated control command to the external apparatus.

11. The method as claimed in claim 10, further comprising:
displaying a user interface (UI) screen for controlling the at least one of the plurality of speakers,
wherein in response to a touch operation of covering the entire UI screen being detected, the transmitting comprises generating a control command for muting the at least one of the plurality of speakers and transmitting the generated control command to the external apparatus.

12. The method as claimed in claim 11, wherein in response to a predetermined motion of the user being recognized within a predetermined distance from the UI screen, the transmitting comprises generating a control command for decreasing a volume level of the at least one of the plurality of speakers and transmitting the generated control command to the external apparatus.

13. The method as claimed in claim 11 or 12, wherein in response to a manipulation of the user for controlling the at least one of the plurality of speakers being received with respect to the UI screen, the transmitting comprises transmitting a control command corresponding to the manipulation to the external apparatus.

14. The method as claimed in any one of claims 11 to 13, further comprising:
sensing location information on the user terminal apparatus,
wherein in response to determining that at least one speaker among the plurality of speakers exists within a predetermined distance from the user terminal apparatus based on the location information on the user terminal apparatus, the displaying comprises automatically displaying a UI screen for controlling the at least one speaker.

15. The method as claimed in any one of claims 10 to 14, further comprising:
recognizing a voice of the user,
wherein in response to a voice of the user for controlling the at least one of the plurality of speakers being recognized, the transmitting comprises transmitting a control command corresponding to the voice to the external apparatus.
